# EUROPÄISCHE PATENTSCHRIFT

(11) **EP 0 562 504 B1**
(45) Veröffentlichungstag und Bekanntmachung des Hinweises auf die Patenterteilung: **02.11.1995**
(21) Anmeldenummer: 93104625.4
(22) Anmeldetag: 22.03.1993
(51) Int. Cl.: C07C 69/24, C07C 67/03, C07C 69/52

(54) **Verfahren zur kontinuierlichen Herstellung von C1-bis C4-Alkylestern höherer Fettsäuren**
Process for the continuous preparation of C1 to C4 alkyl esters of higher fatty acids
Procédé de préparation continue d'esters alkyliques en C1 à C4 d'acides gras supérieurs

(30) Priorität: 26.03.1992 DE 4209779
(43) Veröffentlichungstag der Anmeldung: 29.09.1993
(73) Patentinhaber: OELMÜHLE LEER CONNEMANN GmbH & Co., 26789 Leer (DE)
(72) Erfinder: Connemann, Joosten, Dr. Ing., W-2950 Leer (DE); Krallmann, Anton, W-2953 Rhauderfehn (DE); Fischer, Erich, Dipl.-Ing., W-2956 Moormerland-Warsingsfehn (DE)
(74) Vertreter: Freiherr von Uexküll, Jürgen-Detlev, Dr. Rer. Nat. Dipl.-Chem.

(56) Entgegenhaltungen:
- WO-A-91/05034
- DE-A- 3 020 612

## Beschreibung

Die Erfindung betrifft ein Verfahren zur kontinuierlichen Herstellung von C₁- bis C₄-Alkylestern höherer Fettsäuren aus einer Ölphase, die aus freie Fettsäuren enthaltenden natürlichen Ölen oder Fetten oder C₆- bis C₂₄-Fettsäuretriglyceriden besteht, und C₁- bis C₄-Monoalkoholen durch eine im wesentlichen drucklose, mehrstufige katalytische Umesterung bei Reaktionstemperaturen bis zu 100°C in Gegenwart eines alkalischen Katalysators unter Abtrennung des sich abscheidenden Glycerins, Entfernung der Katalysatorrückstände und Abstrippen niederer Alkohole.

Derartige kontinuierliche Verfahren sind beispielsweise aus der FR-PS 2 560 210 oder DE-OS 34 44 893 bekannt; bei diesem Verfahren erhält man eine bis zu 95%ige bzw. 97%ige Umesterung. Höhere Umesterungen von etwa 98% erhält man auch mit einem mehrstufigen Verfahren gemäß FR-PS 2 577 569 oder gemäß EP-B-0 127 104, welches bei Drucken bis zu 5 bar und in Gegenwart eines flüssigen Schleppmittels durchgeführt wird. Zur Erhöhung der Ausbeute kann das Umesterungsverfahren z.B. gemäß EP-A2-0 198 243 bei höheren Temperaturen von 210 bis 280° und bei 60 bis 80 bar und langen Reaktionszeiten durchgeführt werden. Bei anderen Verfahren werden z.B. gemäß EP-B1-0 192 035 Ionenaustauscher zur Katalysierung der Vorveresterung eingesetzt oder es werden z.B. gemäß OE-PS 386222 mittels Kationenaustauscher die Esterphasen gereinigt.

Allen diesen Verfahren haftet der Nachteil an, daß sie wegen zu hoher Temperaturen oder Drücke oder wegen zu langer Reaktionszeiten und zu großer Apparatevolumina unwirtschaftlich sind oder daß zwar eine Umesterung von bis zu 97,5 bis 98,7% erreicht wird, aber der Anteil an gebundenem und vor allem freiem Glycerin zu hoch ist und eine zu hohe Säurezahl entsteht, weshalb ein erheblicher Aufwand zur Abtrennung des verbliebenen Glycerins und der entstandenen Seifen oder Fettsäuren, beispielsweise durch nachgeschaltete destillative Reinigung, betrieben werden muß.

Man hat auch gemäß DE-PS 39 32 514 vorgeschlagen, Rohrreaktoren mit nachgeschalteten statischen Abscheidern einzusetzen, wobei die Reaktion bei sehr hoher Strömungsgeschwindigkeit durchgeführt wird.

Derartige Verfahren sind jüngst deswegen interessant geworden, weil die Verwendung von Fettsäureestern als Kraftstoff für Dieselmotoren entweder allein oder zusammen mit Methanol und Gasöl geeignet sind, wie es in der DE-OS 37 27 081 mit weiteren dort benannten Literaturstellen beschrieben ist.

Dabei kommt es nach neusten Erfahrungen der Motorenhersteller darauf an, daß nach der Umesterung das fertige Estergemisch für die Verwendung als hochwertiger Dieselkraftstoff einen Gesamtglyceringehalt von unter 0,20 Gew.% und möglichst unter 0,15 Gew.% aufweist, der Gehalt an freiem Glycerin unter 0,01 Gew.% liegt, die Säurezahl nicht höher als 0,2 ist und der Restgehalt an Triglyceriden gegen Null tendiert.

Solche Kennzahlen konnten bisher nur durch eine abschließende destillative Reinigung knapp erreicht werden, während die bisher beschriebenen und realisierten Umesterungsverfahren ohne nachgeschaltete Destillation gerade die Kennzahlen der österreichischen Norm C 1190 erfüllen, nämlich max. 0,25% Gesamtglycerin, 0,03% freies Glycerin und eine Säurezahl von 1,0.

Alle vorgenannten Verfahren beruhen darauf, daß die als Diselkraftstoff geeigneten Ester durch Umesterung (Alkoholyse) aus verschiedensten pflanzlichen Fetten und Ölen mit niederen aliphatischen Alkoholen hergestellt werden. Als Beispiel für Fette bzw. Öle werden Rapsöl, Sonnenblumenöl, Sojaöl, Maisöl, Baumwollöl, Mandelöl, Erdnußöl, Palmöl, Kokosöl, Leinsaatöl, Rizinusöl und insbesondere Rapsöl als vorteilhaft angesehen. Die Umesterung erfolgt mit einem geeigneten einwertigen Alkohol wie Ethanol, Isopropanol, Butanol, auch mehrwertigen Alkoholen wie Trimethylolpropan, insbesondere aber mit Methanol in Anwesenheit eines Umesterungskatalysators, z.B. Metallalkoholaten, -hydriden, -carbonaten, -acetaten oder verschiedenen Säuren, insbesondere aber mit Na-alkoxid, Na- oder K-hydroxid.

Die Erfindung hat sich die Aufgabe gestellt, ein neues, kontinuierlich durchführbares zwei- oder mehrstufiges Verfahren der eingangs erwähnten Art vorzuschlagen, welches neben einer Urnesterung von über 99,2 und bis zu 99,6% wirtschaftlich durchzuführen ist und insbesondere die Abtrennung von Glycerin schnell und nahezu vollständig ermöglicht und ferner eine ausbeutemindernde Seifenbildung weitgehend vermeidet.

Zur Lösung dieser Aufgabe wird ein Verfahren der eingangs gekennzeichneten Art vorgeschlagen, welches gemäß Hauptanspruch ausgebildet ist, wobei bevorzugte Ausführungsformen in den Unteransprüchen erwähnt sind.

Im folgenden soll die Erfindung in Zusammenhang mit dem beiliegenden Fließschema näher erläutert werden. Grundsätzlich wird so vorgegangen, daß in einer ersten Verfahrensstufe eine vorgemischte Mischung aus Ölphase, Monoalkohol und Katalysator bei Reaktionstemperatur von oben in einen als stehende Kolonne ausgebildeten ersten Reaktor mit einer solchen Strömungsgeschwindigkeit eingeführt wird, die niedriger als die Sinkgeschwindigkeit des sich aus dem erhaltenen Reaktionsgemisch I im Gleichstrom abscheidenden Glycerins ist, welches nach einer in der stehenden Kolonne erfolgten Umesterung von etwa 85 bis 90% im Sumpf der Kolonne kontinuierlich abgezogen wird.

Im einzelnen wird hier das pflanzliche Öl, im vorliegenden Falle Rapsöl, aus einem Vorratsbehälter 2 mit einem Durchsatz von 57 l/h durch einen Wärmeaustauscher 4 geführt und auf die Reaktionstemperatur unter 100°C beispielsweise auf eine Reaktionstemperatur zwischen 60 und 75°C erwärmt und zusammen mit dem Alkohol und dem Katalysator einem Mischer 8 zugeführt, der als statischer, jedoch vorzugsweise als dynamischer Mischer ausgebildet ist.

Aus einem Vorratsgefäß 10 mit einem Inhalt von 168 l Methanol und 2 bis 3 kg NaOH werden über eine Leitung 12 Methanol/Alkaligemisch zugeführt, und zwar in einer Menge von 10 l/h, so daß ein 1,4- bis 1,6-facher Überschuß an Methanol mit 0,24 bis 0,36 Gew.% NaOH über die Leitung 12 in den Mischer 8 gelangen.

Dieses Reaktionsgemisch wird in den als stehende Kolonne ausgebildeten ersten Reaktor 14 mit einer solchen Strömungsgeschwindigkeit von oben eingeführt, die niedriger als die Sinkgeschwindigkeit des sich im Gleichstrom abscheidenden Glycerins ist. In dieser Kolonne erfolgt überraschenderweise bereits eine Umesterung von 85 bis 90%. Das sich bei dieser Umesterung bildende Glycerin kann am Boden der Kolonne kontinuierlich oder diskontinuierlich über eine Leitung 16 abgeführt werden.

Das noch etwa 0,5 Gew.% gelöstes und zusätzlich dispergiertes Glycerin enthaltende Reaktionsgemisch I, das aus niederen Alkylestern, Monoalkoholen, Katalysator, Öl und teilumgesetzten Öl besteht, wird kurz oberhalb des Bodens der Kolonne 14 über eine Leitung 18 zur weiteren Umesterung in einen zweiten als Rührreaktor ausgebildeten Reaktor 20 überführt und bei einer Verweilzeit von 2 Minuten bis zu 2 Stunden und vorzugsweise einer Stunde und bei Reaktionstemperaturen von 60 bis 80°C, bis zu etwa 95% bis 97% weiter umgeestert.

Das hierbei erhaltene Reaktionsgemisch II wird dann in einer Trennstufe von weiterem Glycerin befreit, wobei die Glycerinabtrennung auf zweierlei Weise erfolgen kann. Entweder wird das Reaktionsgemisch II von dem Rührreaktor 20 über eine Leitung 22 mittels eines Wäremaustauschers 24 bei einer Temperatur von 20 bis 40°C und vorzugsweise 25 bis 30°C einem Separator 26 zugeführt oder es wird in dem Wärmeaustauscher 24 auf eine Temperatur von 70 bis 90°C gebracht und unter Zugabe von 0,25 bis 10 Gew.% einer heißen wäßrigen Extraktionslösung in den Separator 26 eingeleitet. Dieser Hochleistungsseparator ist vorzugsweise als Tellerseparator ausgebildet, der nicht nur die Phasen unterschiedlicher Dichte trennt, sondern gleichzeitig eine Kurzzeit-Extraktion der leichten Esterphase mit zusätzlich eingeführter wäßriger Phase erlaubt, um das die Gleichgewichtsverschiebung hindernde gelöste Glycerin nahezu vollständig zu extrahieren, ohne daß eine zusätzliche Verseifung eintritt, und wird mit 50 l/h des Reaktionsgemisches II beschickt.

Ein Teil der leichten Phase kann auf den vorgeschalteten Rührreaktor im Kreislauf zurückgeführt werden, um auch schon dort die Glycerin-Konzentration deutlich zu ermäßigen.

Wenn das Reaktionsgemisch II bei einer Temperatur von 20 bis 40°C in den Separator gegeben wird, werden etwa 1 Gew.% weiteres Glycerin entfernt, während bei Zuführung des heißen Reaktionsgemisches II unter Zusatz einer heißen wäßrigen Extraktionslösung 1 bis 2,5 Gew.% weiteres Glycerin entfernt werden und damit die Voraussetzung für eine nachfolgende zusätzliche Umesterung geschaffen wird.

In einer weiteren Verfahrensstufe wird nämlich das derart erhaltene Reaktionsgemisch II bei Reaktionstemperatur und im Kreuzstrom unter neuer Zufuhr von weiteren C₁- bis C₄-Monoalkoholen und Katalysator einem als stehende Kolonne ausgebildeten zweiten Reaktor mit einer der ersten Verfahrensstufe entsprechenden Strömungsgeschwindigkeit eingeführt.

Hierbei wird im einzelnen das Reaktionsgemisch II bzw. Umesterungsprodukt mit einer Geschwindigkeit von 50 l/h über eine Leitung 28 einem weiteren Wärmeaustauscher 30 zugeführt und mit einer Temperatur von 70 bis 90°C in eine Mischpumpe 34 geführt, die gleichzeitig aus dem den Alkohol und Natronlauge enthaltenden Vorratsgefäß 10 über die Leitung 32 mit einer Geschwindigkeit von 1,5 bis 3 l/h eine Mischung aus 0,54 bis 0,11% NaOH und Alkohol in einem 0,2- bis 0,4-fachen Überschuß zugeführt wird. Diese Mischung wird über einen zweiten ebenfalls als stehende Kolonne ausgebildeten Reaktor 36 geführt, in welchem die weitere Umesterung mit einem Ergebnis von 98,5 bis 99% durchgeführt wird. Auch hierbei ist die Strömungsgeschwindigkeit derart bemessen, daß sie niedriger als die Sinkgeschwindigkeit des sich aus dem Reaktionsgemisch abscheidenden Glycerins ist. Das am Boden der Kolonne abgezogene Reaktionsgemisch III gelangt über eine Leitung 38 in einen zweiten Rührreaktor 40 und wird dort bei Temperaturen von 60 bis 80°C und einer Verweilzeit von 0,5 bis 2 Stunden und vorzugsweise bei einer Temperatur von 65 bis 68°C und einer Verweilzeit von einer Stunde weiter umgeestert.

Das aus diesem Rührreaktor erhaltene Produkt wird über eine Leitung 42 einem Wärmeaustauscher 44 zugeführt und dann bei Temperaturen von 70 bis 90°C in einer weiteren Trennstufe über eine Leitung 46 einem weiteren Separator 48 zugeführt, der analog dem ersten Separator 26 ausgebildet ist. Dieser Separator wird analog der einen Variante der ersten Trennstufe mit einer heißen wäßrigen Pufferlösung oder wäßrigen Extraktionslösung aus einem Vorratsbehälter 50 über die Leitung 52 gespeist. Hierbei werden Natronlauge, etwa gebildete Seife und Glycerin mit dem Wasser abgetrennt, wobei das nunmehr erhaltene Reaktionsgemisch IV zur weiteren Reinigung über eine Leitung 54 abgeführt wird, während ein Teil dieses Reaktionsproduktes über eine Leitung 56 in den zweiten Rührreaktor 40 zurückgeführt wird. Hierdurch wird erreicht, daß auch im zweiten Rührreaktor 40 die Konzentration des gelösten Glycerins anteilig herabgesetzt wird, um das Reaktionsgleichgewicht zu verschieben und einen höheren Umesterungsgrad zu erreichen. Das der weiteren Reinigung zugeführte Reaktionsprodukt IV hat einen Umesterungsgrad von 99,2% bis 99,6% und enthält nur noch etwa 500 bis 800 ppm Seife, außerdem etwas Methanol und Wasser.

Zur Entfernung des restlichen Methanols aus dem Reaktionsprodukt IV wird dieser dann über einen Wärmeaustauscher 57 über eine Leitung 58 einer Stripping-Kolonne 60 zugeführt, die beheizt ist und unter leichtem Vakuum betrieben wird, so daß die Esterphase beim Verlassen derselben einen Methanolgehalt von deutlich unter 0,1% aufweist. Das verdampfte Methanol wird einem nicht eingezeichneten Kondensationssystem, an welches auch sämtliche Kolonnen und Rührbehälter angeschlossen sind, zugeführt, kondensiert und über eine ausreichende Rektifikation im Kreislauf auf den Katalysator-Ansatzbehälter 10 zurückgeführt.

Das Reaktionsgemisch IV wird anschließend zur ersten Waschstufe geleitet, und zwar über einen Wärmeaustauscher 62 auf eine Temperatur von 80 bis 90°C eingestellt und mit etwa 2 bis 10 l/h Wasser von 80 bis 90°C über eine Leitung 63 einem weiteren Separator 64 zugeführt, in welchem Katalysatorreste, Wasser, Seife und restliches Glycerin entfernt werden. Das zur zweiten Waschstufe weitergeleitete Reaktionsprodukt enthält nur noch 150 bis 300 ppm Seife und wird aus einem Vorratsbehälter 67 nochmals mit 2 bis 10 l/h Wasser bei 70 bis 90°C gewaschen und über eine Leitung 65 in einem weiteren Separator 66 von Glycerin, Wasser und Seife weitgehend befreit und gelangt mit einem Seifengehalt von nunmehr nur noch 15 bis 30 ppm über eine Leitung 68 in eine bei 110 bis 120°C und 0,9 bar betriebene Trocknungsvorrichtung 70, von wo es über eine Leitung 72 und einen Kühler 74 noch einem Filter 76 zugeführt wird, bevor es als Endprodukt in einen Lagertank gelangt.

Die leichten Phasen aus den Separatoren 64 und 66 werden über Leitungen 69 bzw. 69' der jeweils vorherigen Stufe zurückgeführt.

Zur etwaigen Erzielung von Wintertauglichkleit des Dieselkraftstoffes ist es je nach Reaktionsführung und Ausgangsmaterial sinnvoll, das Reaktionsgemisch IV auf etwa -25°C abzukühlen, um in einem weiteren Separator bei einer Arbeitstemperatur von -12 bis -15°C die höherschmelzenden Alkylester zu entfernen. Ein erster Beitrag zu besserer Wintertauglichkeit besteht schon darin, daß die anfängliche Alkoholyse mit einem Gemisch von Methanol und Ethanol durchgeführt wird.

Vorzugsweise werden die Kontaktzeiten in den Wasch- und Trennstufen zur Vermeidung von Hydrolyseeffekten mit Hilfe der Tellerseparatoren auf 1 bis 20 Sekunden beschränkt. Letztlich kann der Rührreaktor zur Behandlung verschiedener Ausgangsmaterialien auf verschiedene Verweilzeiten eingestellt werden.

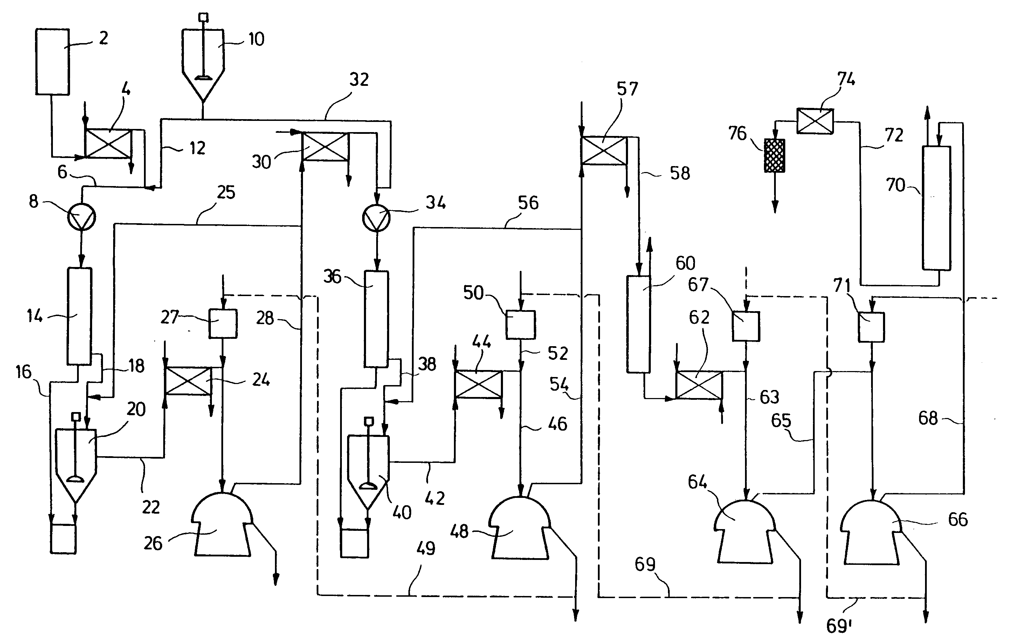

## Patentansprüche

1. Verfahren zur kontinuierlichen Herstellung von C₁- bis C₄-Alkylestern höherer Fettsäuren aus einer Ölphase, die aus freie Fettsäuren enthaltenden natürlichen Ölen oder Fetten oder C₆- bis C₂₄-Fettsäuretriglyceriden besteht, und C₁- bis C₄-Monoalkoholen durch eine im wesentlichen drucklose, mehrstufige katalytische Umesterung bei Reaktionstemperaturen bis zu 100°C in Gegenwart eines alkalischen Katalysators unter Abtrennung des sich abscheidenden Glycerin, Entfernung der Katalysatorrückstände und Abstrippen niederer Alkohole, **dadurch gekennzeichnet**, daß
a) in einer ersten Verfahrensstufe eine statisch oder dynamisch vorgemischte Mischung aus Ölphase, Monoalkohol und Katalysator bei Reaktionstemperatur von oben in einen als stehende Kolonne ausgebildeten ersten Reaktor mit einer solchen Strömungsgeschwindigkeit eingeführt wird, die niedriger als die Sinkgeschwindigkeit des sich aus den erhaltenen Reaktionsgemisch I abscheidenden Glycerins ist, welches nach einer in der stehenden Kolonne erfolgten Umesterung von etwa 85 bis 90% im Sumpf der Kolonne kontinuierlich abgezogen wird, worauf
b) das noch etwa 0,5 Gew.% gelöstes und zusätzlich noch mehr dispergiertes Glycerin enthaltende Reaktionsgemisch I, das aus niederen Alkylestern, Monoalkoholen, Katalysator, Öl und teilumgesetzten Öl besteht zur weiteren Umesterung in einen zweiten als Rührreaktor ausgebildeten Reaktor überführt und bei einer Verweilzeit von 2 Minuten bis zu 2 Stunden bei Reaktionstemperaturen von 60 bis 80°C bis zu etwa 95% bis 97% weiter umgeestert wird, worauf
c) das derart erhaltene Reaktionsgemisch II in einer ersten Trennstufe
i) mit einer Temperatur von 20 bis 40°C, oder
ii) mit einer Temperatur von 70 bis 90°C unter Zugabe von 0,25 bis 10 Gew.% einer heißen wäßrigen Extraktionslösung
in einem besonderen Separator durch eine Kurzzeitwäsche mit einer Extraktionslösung von 1 bis 2,5 Gew.% weiteren Glycerins befreit wird, worauf
d) in einer weiteren Verfahrensstufe das derart erhaltene Reaktionsgemisch II bei Reaktionstemperatur und unter Zufuhr von weiteren C₁- bis C₄-Monoalkoholen und Katalysator einem als stehende Kolonne ausgebildeten zweiten Reaktor mit einer der ersten Verfahrensstufe entsprechenden Strömungsgeschwindigkeit eingeführt und das Reaktionsprodukt III
e) in einen weiteren Rührreaktor bei Temperaturen von 60 bis 80°C und einer Verweilzeit von 0,5 bis 2 Stunden weiter umgeestert wird, worauf das bis zu 99,2% bis 99,6% umgeesterte Reaktionsprodukt IV
f) in einer zweiten Trennstufe bei 70 bis 90°C unter Zugabe von 0,25 bis 10 Gew.% einer wäßrigen Extraktionslösung bzw. einer Pufferlösung einem weiteren Separator zugeführt wird und von restlichem Glycerin, gebildeten Seifen und Katalysator befreit wird, worauf
g) das so gereinigte Reaktionsgemisch IV durch Strippen von den niederen Alkoholen befreit, mehrfach mit geeigneten Extraktions- und Waschlösungen gewaschen und dann getrocknet wird.

2. Verfahren nach Anspruch 1, **dadurch gekennzeichnet**, daß als Separator Tellerseparatoren verwendet werden, die eine zusätzliche Kurzzeitbehandlung der kontinuierlichen Phase bzw. Extraktion der kontinuierlichen oder dispergierten Phase mit einem Medium geeigneter Dichte ermöglichen.

3. Verfahren nach Anspruch 1 bis 2, **dadurch gekennzeichnet**, daß als geeignetes Medium wäßrige Lösungen im sauren oder basischen pH-Bereich bzw. abgepufferte Lösungen verwendet werden.

4. Verfahren nach Anspruch 1, **dadurch gekennzeichnet**, daß ein Teil der im Separator deglycerinierten leichten Esterphase im Kreislauf auf den Rührreaktor zurückgeführt wird, um die vorhandene Glycerin-Konzentration weiter zu verringern und damit das Reaktionsgleichgewicht zu verschieben.

5. Verfahren nach Anspruch 1, **dadurch gekennzeichnet**, daß die Kontaktzeiten in den Wasch- und Trennstufen zur Vermeidung von Hydrolyseeffekten mit Hilfe der Tellerseparatoren auf 1 bis 20 Sekunden beschränkt werden.

6. Verfahren nach Anspruch 1, **dadurch gekennzeichnet**, daß die Wasch- und Extraktionsflüssigkeiten im Gegenstrom zur Esterphase durch die Waschstufen und nach Seifenabtrennung auch auf die Trennstufen geführt werden.

7. Verfahren nach Anspruch 1 bis 6, **dadurch gekennzeichnet**, daß das Reaktionsgemisch II nach Zuführen in den als stehende Kolonne ausgebildeten dritten Reaktor in einen weiteren Reaktor eingeleitet und bei Reaktionstemperatur weiter umgeestert wird.

8. Verfahren nach Anspruch 1 bis 7, **dadurch gekennzeichnet**, daß die zugeführte Alkoholmenge in der ersten Umesterungsstufe 120 bis 180% und in der zweiten Umesterungsstufe 20 bis 60% der stöchiometrisch erforderlichen Menge beträgt.

9. Verfahren nach Anspruch 1 bis 8, **dadurch gekennzeichnet**, daß die zugeführte Alkoholmenge in der ersten umesterungsstufe 150% und in der zweiten Umesterungsstufe 30% der stöchiometrisch erforderlichen Menge beträgt.

10. Verfahren nach Anspruch 1 bis 9, **dadurch gekennzeichnet**, daß die Katalysator-Konzentration in der ersten und der zweiten Verfahrensstufe unterschiedlich ist und in der zweiten Stufe das 1,1 bis 3,0-fach der ersten Verfahrensstufe beträgt.

11. Verfahren nach Anspruch 1 bis 10, **dadurch gekennzeichnet**, daß die Katalysator-Konzentration in der zweiten Verfahrensstufe das 1,5-fache der ersten Verfahrensstufe beträgt.

12. Verfahren nach Anspruch 1 bis 11, **dadurch gekennzeichnet**, daß als wäßrige Extraktionslösung zur Abtrennung weiteren Glycerins im Separator eine alkalische Pufferlösung mit einem pH-Wert von 8 bis 10 verwendet wird.

13. Verfahren nach Anspruch 1 bis 12, **dadurch gekennzeichnet**, daß man das aus dem Separator erhaltene Reaktionsgemisch II oder IV in einer Menge von 40 bis 70 Gew.% in den vorgeschalteten Reaktor rückführt.

14. Verfahren nach Anspruch 1 bis 13, **dadurch gekennzeichnet**, daß das nach dem Abstrippen erhaltene Reaktionsgemisch IV bis auf -25°C gekühlt und in einem Temperaturbereich von - 12° bis -15°C in einem Separator von höherschmelzenden Alkylestern befreit wird.

## Claims

1. Method for the continuous preparation of C₁-to C₄-alkyl esters of higher fatty acids from an oil phase, which consists of natural oils or fats containing free fatty acids or consists of C₆- to C₂₄-fatty acid triglycerides, and C₁- to C₄-monoalcohols by means of substantially pressure-less, multistage, catalytic transesterification at reaction temperatures of up to 100°C in the presence of an alkaline catalyst with separation of the glycerine which is precipitated, removal of the catalyst residues and stripping off of lower alcohols, characterised in that
a) in a first method stage a mixture, which is premixed statically or dynamically and consists of an oil phase, monoalcohol and catalyst, at the reaction temperature, is introduced from above into a first reactor, which is formed as an upright column, at a velocity of flow which is such that it is lower than the settling velocity of the glycerine which is precipitated from the reaction mixture I obtained and is continuously drawn off at the sump of the column after transesterification of approximately 85 to 90% has occurred in the upright column, whereupon
b) the reaction mixture I, which still contains approximately 0.5% by weight dissolved and, in addition, even more dispersed glycerine and consists of lower alkyl esters, monoalcohols, catalyst, oil and partly reacted oil, is transferred, for the purpose of further transesterification, into a second reactor formed as a stirring reactor and undergoes further transesterification of up to approximately 95% to 97% with a dwell time of 2 minutes to up to 2 hours at reaction temperatures of 60 to up to 80°C, whereupon
c) the reaction mixture II thus obtained is freed of 1 to 2.5% by weight further glycerine in a first separating stage
i) at a temperature of 20 to 40°C, or
ii) at a temperature of 70 to 90°C with addition of 0.25 to 10% by weight of a hot aqueous extraction solution
in a special separator by means of a short-time wash with an extraction solution, whereupon
d) in a further method stage the reaction mixture II thus obtained is introduced, at the reaction temperature and with supply of further C₁- to C₄-monoalcohols and catalyst, to a second reactor, which is formed as an upright column, at a velocity of flow which corresponds to that of the first method stage, and the reaction product III
e) undergoes further transesterification in a further stirring reactor at temperatures of 60 to 80°C and with a dwell time of 0.5 to 2 hours, whereupon the reaction product IV which has undergone transesterification of up to 99.2% to 99.6%
f) is supplied to a further separator in a second separating stage at 70 to 90°C with addition of 0.25 to 10% by weight of an aqueous extraction solution or a buffer solution and is freed of residual glycerine, soaps which have formed and catalyst, whereupon
g) the thus purified reaction mixture IV is freed of the lower alcohols by stripping, is washed several times with suitable extraction and washing solutions and then is dried.

2. Method according to claim 1, characterised in that disc separators are used as the separator, which disc separators render possible additional short-time treatment of the continuous phase or extraction of the continuous or dispersed phase with a medium of suitable density.

3. Method according to claim 1 to 2, characterised in that aqueous solutions in the acidic or basic pH-range or buffered solutions are used as the suitable medium.

4. Method according to claim 1, characterised in that a part of the light ester phase, from which glycerine has been removed in the separator, is recirculated to the stirring reactor in order to reduce further the glycerine concentration which is present and with that to displace the equilibrium of reaction.

5. Method according to claim 1, characterised in that the contact times in the washing and separating stages are limited to 1 to 20 seconds with the aid of the disc separators in order to avoid effects of hydrolysis.

6. Method according to claim 1, characterised in that the washing and extraction fluids are conducted in countercurrent to the ester phase through the washing stages and after soap separation also to the separating stages.

7. Method according to claim 1 to 6, characterised in that the reaction mixture II after being supplied to the third reactor, which is formed as an upright column, is passed into a further reactor and undergoes further transesterification at the reaction temperature.

8. Method according to claim 1 to 7, characterised in that the quantity of alcohol supplied in the first transesterification stage amounts to 120 to 180% and in the second transesterification stage amounts to 20 to 60% of the stoichiometrically required quantity.

9. Method according to claim 1 to 8, characterised in that the quantity of alcohol supplied in the first transesterification stage amounts to 150% and in the second transesterification stage amounts to 30% of the stoichiometrically required quantity.

10. Method according to claim 1 to 9, characterised in that the catalyst concentration in the first method stage differs from that in the second method stage and in the second stage amounts to 1.1 to 3.0 times that of the first method stage.

11. Method according to claim 1 to 10, characterised in that the catalyst concentration in the second method stage amounts to 1.5 times that of the first method stage.

12. Method according to claim 1 to 11, characterised in that an alkaline buffer solution with a pH-value of 8 to 10 is used as the aqueous extraction solution for the separation of further glycerine in the separator.

13. Method according to claim 1 to 12, characterised in that 40 to 70 % by weight of the reaction mixture II or IV obtained from the separator is returned to the reactor upstream thereof.

14. Method according to claim 1 to 13, characterised in that the reaction mixture IV obtained after the stripping-off is cooled to -25°C and is freed of higher melting alkyl esters in a separator in a temperature range of -12° to -15°C.

## Revendications

1. Procédé de préparation continue d'esther alkylique en C₁ à C₄ d'acides gras supérieurs à partir d'une phase huileuse qui est constituée d'huile naturelle ou de graisse contenant des acides gras libres ou de triglycéride d'acides gras en C₆ à C₂₄, et de mono-alcools en C₁ à C₄ par une transesthérification catalytique essentiellement sans pression en plusieurs étapes à des températures de réaction pouvant aller jusqu'a 100 °C, en présence d'un catalyseur alcalin, par séparation de la glycérine précipitant, élimination des résidus de catalyseur et strippage d'alcools inférieurs, caractérisé en ce que
a) dans une première étape de procédé un mélange prémélangé statiquement ou dynamiquement de phase huileuse, de mono-alcool de catalyseur, est introduit à la température de réaction, à partir du haut, dans un premier réacteur conçu en tant que colonne verticale, avec une vitesse d'écoulement qui est inférieure à la vitesse de descente de la glycérine précipitant à partir du mélange de réaction I obtenu, qui après une transesthérification, effectuée dans la colonne verticale, de 65 à 90 % environ, est soutirée en continu dans le fond de la colonne, après quoi
b) le mélange de réaction I contenant encore environ 0,5 % en poids de glycérine dissoute et en supplément encore plus dispersée, qui est constitué d'esthers alkyliques inférieurs, de mono-alcools, de catalyseur, d'huile et d'huile en partie transformée, est transféré pour la poursuite de la transesthérification dans un second réacteur, conçu en tant que réacteur à agitation, et est transesthérifié pendant un temps de séjour de 2 minutes à 2 heures, à des températures de réaction comprises entre 60 et 80 °C, jusqu'à environ 95 % à 97 %, après quoi
c) le mélange de réaction II ainsi obtenu est encore libéré de la glycérine dans une première étape de séparation
i) à une température de 20 à 40 °C, ou
ii) à une température de 70 à 90 °C par addition de 0,25 à 10 % en poids d'une solution d'extraction aqueuse chaude
dans un séparateur particulier, par un lavage de courte durée avec une solution d'extraction de 1 à 2,5 % en poids, après quoi
d) dans une autre étape de procédé, le mélange de réaction II ainsi obtenu est introduit, à la température de réaction et par apport d'autres mono-alcools en C₁ à C₄ et catalyseur dans un second réacteur, conçu en colonne verticale, avec une vitesse d'écoulement correspondant à la première étape de procédé, et le produit de réaction III
e) est encore transesthérifié dans un autre réacteur à agitation à des températures comprises entre 60 et 80 °C et avec un temps de séjour de 0,5 à 2 heures, après quoi le produit de réaction IV, transesthérifié jusqu'à 99,2 % à 99,6 %
f) est envoyé, dans une deuxième étape de séparation, entre 70 et 90 °C, avec addition de 0,25 à 10 % en poids d'une solution d'extraction aqueuse ou d'une solution tampon, à un autre séparateur et est libéré de la glycérine restante des savons qui se sont formés et du catalyseur, après quoi
g) le mélange de réaction IV épuré est libéré par strippage des alcools inférieurs, lavé plusieurs fois avec des solutions d'extraction et de lavage appropriées, puis séché.

2. Procédé selon la revendication 1, caractérisé en ce qu'on utilise comme séparateur, des séperateurs à plateau qui permettent un traitement de courte durée supplémentaire de la phase continue ou extraction de la phase continue ou dispersée avec un fluide de densité approprié.

3. Procédé selon les revendications 1 et 2, caractérisé en ce que comme fluide approprié on utilise des solutions aqueuses de pH acide ou basique ou des solutions tamponnées.

4. Procédé selon la revendication 1, caractérisé en ce qu'une partie de la phase esther légère déglycérinée dans le séparateur est renvoyé dans le circuit au réacteur à agitation, afin de réduire encore la concentration de glycérine et de déplacer ainsi l'équilibre de la réaction.

5. Procédé selon la revendication 1, caractérisé en ce que les temps de contact dans les étapes de lavage et de séparation sont limités, à l'aide des séparateurs à plateau, à 1 à 20 secondes, afin d'éviter des effets d'hydrolyse.

6. Procédé selon la revendication 1, caractérisé en ce que le liquide de lavage et d'extraction sont envoyés en contre-courant à la phase esther, à travers les étapes de lavage et après séparation des savons, également aux étapes de séparation.

7. Procédé selon les revendications 1 à 6, caractérisé en ce que le mélange de réaction II, après envoi au troisième réacteur conçu en tant que colonne verticale, est introduit dans un autre réacteur et est encore transesthérifié à la température de réaction.

8. Procédé selon les revendications 1 à 7, caractérisé en ce que la quantité d'alcool alimenté représente, dans la première étape de transesthérification, de 120 à 180 % et dans la seconde étape de transesthérification, de 20 à 60 % de la quantité stochiométriquement nécessaire.

9. Procédé selon les revendications 1 à 8, caractérise en ce que la quantité d'alcool alimenté représente, dans la première étape de transesthérification 150 % et dans la seconde étape de transesthérification, 30 % de la quantité stochiométriquement nécessaire.

10. Procédé selon les revendications 1 à 9, caractérisé en ce que la concentration de catalyseur dans la première et dans la seconde étape de procédé est différente et représente, dans la seconde étape, de 1,1 à 3 fois la première étape de procédé.

11. Procédé selon les revendications 1 à 10, caractérisé en ce que la concentratrion de catalyseur dans la seconde étape de procédé représente 1,5 fois la première étape de procédé.

12. Procédé selon les revendications 1 à 11, caractérisé en ce que comme solution d'extraction aqueuse pour la séparation complémentaire de glycérine dans le séparateur, on utilise une solution tampon alcaline d'un pH compris entre 8 et 10.

13. Procédé selon les revendications 1 à 12, caractérisé en ce qu'on recycle le mélange de réaction II ou IV, obtenu à partir du séparateur, dans une quantité de 40 à 70 % en poids, dans le réacteur amont.

14. Procédé selon les revendications 1 à 13, caractérisé en ce que le mélange de réaction IV, obtenu après strippage, est refroidi jusqu'à -25 °C et est libéré des esthers alkyliques à haut point de fusion, dans une plage de température allant de -12° à -15 °C, dans un séparateur.
